Europäisches Patentamt
European Patent Office
Office européen des brevets

(11) Veröffentlichungsnummer: **0 351 646**
**A2**

(12) **EUROPÄISCHE PATENTANMELDUNG**

(21) Anmeldenummer: **89112301.0**

(22) Anmeldetag: **06.07.89**

(51) Int. Cl.⁴: **C07D 249/08 , A01N 43/653 , C07D 303/08 , C07C 49/567**

(30) Priorität: **19.07.88 DE 3824434**

(43) Veröffentlichungstag der Anmeldung: **24.01.90 Patentblatt 90/04**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Lantzsch, Reinhard, Dr.**
**Am Buschhäuschen 51**
**D-5600 Wuppertal 1(DE)**
Erfinder: **Kysela, Ernst, Dr.**
**Virchowstrasse 14**
**D-5060 Bergisch-Gladbach 1(DE)**
Erfinder: **Büchel, Karl Heinz, Prof. Dr.**
**Dabringhausener Strasse 42**
**D-5093 Burscheid(DE)**
Erfinder: **Dutzmann, Stefan, Dr.**
**Leinenweberweg 33**
**D-4000 Düsseldorf 13(DE)**
Erfinder: **Brandes, Wilhelm, Dr.**
**Eichendorffstrasse 3**
**D-5653 Leichlingen(DE)**
Erfinder: **Hänssler, Gerd, Dr.**
**Am Arenzberg 58a**
**D-5090 Leverkusen 3(DE)**

(54) **Hydroxyalkinyl-azolyl-Derivate.**

(57) Neue Hydroxyalkinyl-azolyl-Derivate der Formel

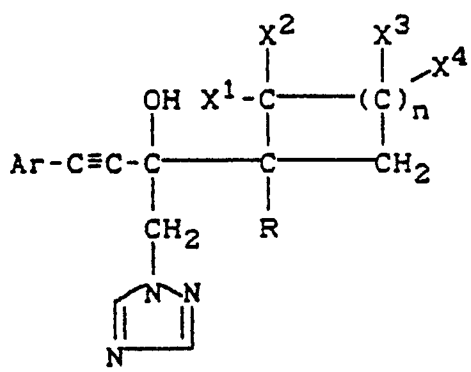

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht
und

EP 0 351 646 A2

n für 0 oder 1 steht,

sowie deren Säureadditions-Salze und Metallsalz-Komplexe,

mehrere Verfahren zur Herstellung der neuen Stoffe und deren Verwendung als Fungizide.

Neue Zwischenprodukte, Verfahren zu deren Herstellung und deren Verwendung zur Synthese von Hydroxyalkinyl-azolyl-Derivaten der Formel (I).

## Hydroxyalkinyl-azolyl-Derivate

Die vorliegende Erfindung betrifft neue Hydroxyalkinylazolyl-Derivate, mehrere Verfahren zu deren Herstellung und deren Verwendung als Fungizide.

Es ist bereits bekannt geworden, daß bestimmte Hydroxyalkinyl-azolyl-Derivate fungizide Eigenschaften besitzen (vgl. EP-OS 0 052 424 und EP-OS 0 108 995). So kann zum Beispiel 1-(4-Chlorphenyl)-3-(1,2,4-triazol-1-yl-methyl)-4,4-dimethyl-pent-1-in-3-ol zur Bekämpfung von Pilzen eingesetzt werden. Die Wirksamkeit dieses Stoffes ist gut, läßt aber bei niedrigen Aufwandmengen in manchen Fällen zu wünschen übrig.

Es wurden nun neue Hydroxyalkinyl-azolyl-Derivate der Formel

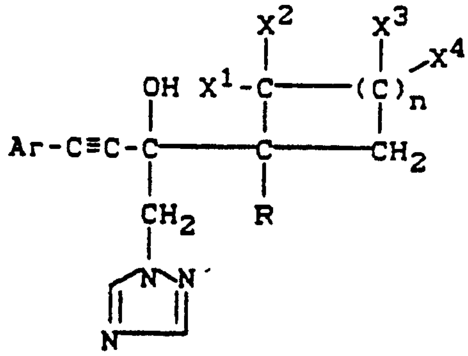

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht,
und
n für 0 oder 1 steht,
sowie deren Säureadditions-Salze und Metallsalz-Komplexe gefunden.

Die erfindungsgemäßen Stoffe enthalten mindestens zwei asymmetrisch substituierte Kohlenstoffatome. Sie können daher in optischen Isomerenformen anfallen. Die Erfindung betrifft sowohl die einzelnen Isomeren als auch deren Gemische.

Weiterhin wurde gefunden, daß man Hydroxyalkinyl-azolyl-Derivate der Formel (I) sowie deren Säureadditions-Salze und Metallsalz-Komplexe erhält, wenn man

a) Oxirane der Formel

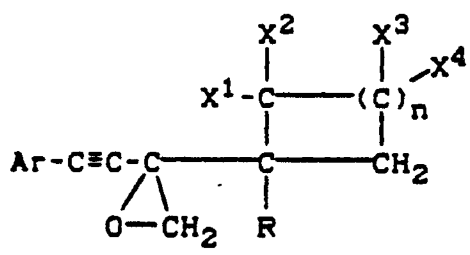

(II)

in welcher
Ar, R, $X^1$, $X^2$, $X^3$, $X^4$ und n die oben angegebene Bedeutung haben,
mit 1,2,4-Triazol der Formel

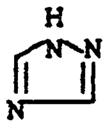 (III)

in Gegenwart eines Säurebindemittels und in Gegenwart eines Verdünnungsmittels umsetzt,
oder

b) Acetylen-Derivate der Formel

Ar-C≡CH (IV)

in welcher
Ar die oben angegebene Bedeutung hat,
mit Azolylmethyl-ketonen der Formel

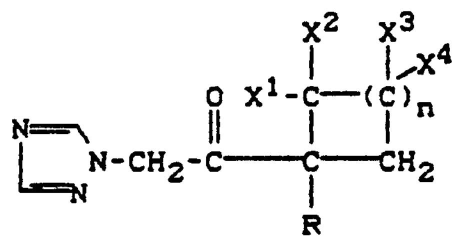 (V)

in welcher
R, $X^1$, $X^2$, $X^3$, $X^4$ und n die oben angegebene Bedeutung haben,
in Gegenwart einer Base und in Gegenwart eines Verdünnungsmittels sowie gegebenenfalls in Gegenwart eines Phasentransfer-Katalysators umsetzt, und gegebenenfalls anschließend an die so erhaltenen Verbindungen der Formel (I) eine Säure oder ein Metallsalz addiert.

Schließlich wurde gefunden, daß die neuen Hydroxy-alkinyl-azolyl-Derivate der Formel (I) sowie deren Säureadditions-Salze und Metallsalz-Komplexe sehr gute fungizide Eigenschaften besitzen.

Überraschenderweise zeigen die erfindungsgemäßen Stoffe eine bessere fungizide Wirksamkeit als das 1-(4-Chlorphenyl)-3-(1,2,4-triazol-1-yl-methyl)-4,4-dimethyl-pent-1-in-3-ol, welches ein konstitutionell ähnlicher, vorbekannter Wirkstoff gleicher Wirkungsrichtung ist.

Die erfindungsgemäßen Hydroxyalkinyl-azolyl-Derivate sind durch die Formel (I) allgemein definiert. Bevorzugt sind diejenigen Verbindungen, in denen

Ar für Phenyl steht, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy,
R für Alkyl mit 1 bis 4 Kohlenstoffatomen steht oder für Phenyl steht, das einfach bis dreifach, gleichartig oder veschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy;
oder
R für Benzyl steht, das im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio

mit 1 bis 4 Kohlenstoffatomen, Halogenalkyl mit 1 oder 2 Kohlenstoffatomen und 1 bis 5 gleichen oder verschiedenen Halogenatomen, Halogen alkoxy mit 1 oder 2 Kohlenstoffatomen und 1 bis 3 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy;
$X^1$ für Fluor, Chlor oder Brom steht,
$X^2$ für Wasserstoff, Fluor, Chlor oder Brom steht,
$X^3$ für Wasserstoff, Fluor, Chlor oder Brom steht,
$X^4$ für Wasserstoff, Fluor, Chlor oder Brom steht, und
n für 0 oder 1 steht.

Besonders bevorzugt sind diejenigen Verbindungen der Formel (I), in denen
Ar für Phenyl steht, das einfach bis dreifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, 1-Methoximino-1-ethyl, Ethoximinomethyl, gegebenenfalls durch Fluor, Chlor und/oder Methyl substituiertes Phenyl oder gegebenenfalls durch Fluor, Chlor und/oder Methyl substituiertes Phenoxy;
R für Methyl, Ethyl, Isopropyl, tert.-Butyl steht, oder für gegebenenfalls einfach bis zweifach, gleich oder verschieden durch Fluor, Chlor oder Methyl substituiertes Phenyl steht, oder für Benzyl steht, das im Phenylteil einfach oder zweifach, gleichartig oder verschieden substituiert sein kann durch Fluor, Chlor und/oder Methyl,
$X^1$ für Fluor oder Chlor steht,
$X^2$ für Wasserstoff, Fluor oder Chlor steht,
$X^3$ für Wasserstoff, Fluor oder Chlor steht,
$X^4$ für Wasserstoff, Fluor oder Chlor steht und
n für 0 oder 1 steht.

Ganz besonders bevorzugt sind diejenigen Verbindungen der Formel (I), in denen
Ar für Phenyl steht, das einfach bis dreifach, gleichartig oder verschieden substituiert sein kann durch Fluor, Chlor, Brom, Methyl, Ethyl, Isopropyl, tert.-Butyl, Methoxy, Methylthio, Trifluormethyl, Trifluormethoxy, Trifluormethylthio, Methoximinoethyl, 1-Methoximino-1-ethyl, Ethoximinomethyl, gegebenenfalls durch Fluor, Chlor und/oder Methyl substituiertes Phenyl oder gegebenenfalls durch Fluor, Chlor und/oder Methyl substituiertes Phenoxy;
R für Methyl, Ethyl, gegebenenfalls einfach oder zweifach durch Fluor, Chlor und/oder Methyl substituiertes Phenyl oder für Benzyl steht,
$X^1$ für Fluor oder Chlor steht,
$X^2$ für Wasserstoff, Fluor oder Chlor steht,
$X^3$ für Wasserstoff, Fluor oder Chlor steht,
$X^4$ für Wasserstoff, Fluor oder Chlor steht,
und
n für 0 oder 1 steht.

Bevorzugte erfindungsgemäße Verbindungen sind auch Additionsprodukte aus Säuren und denjenigen Hydroxyalkinyl-azolyl-Derivaten der Formel (I), in denen Ar, R, $X^1$, $X^2$, $X^3$, $X^4$ und n die Bedeutungen haben, die bereits vorzugsweise für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Hydroxyalkinyl-azolyl-Derivaten der Formel (I), in denen Ar, R, $X^1$, $X^2$, $X^3$, $X^4$ und n die Bedeutungen haben, die bereits vorzugsweise für diese Reste bzw. diesen Index genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Hydroxyalkinyl-azolyl-Derivate der Formel (I) seien die in der folgenden Tabelle 1 aufgeführten Stoffe genannt:

**Tabelle 1**

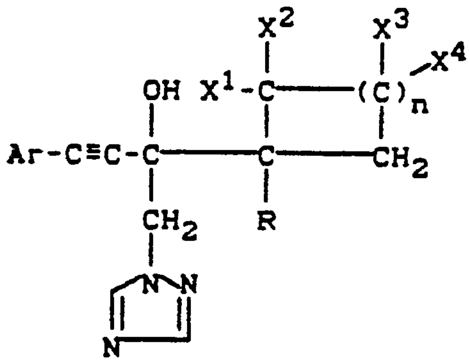

(I)

| Ar | $X^1$ | $X^2$ | $X^3$ | $X^4$ | n | R |
|---|---|---|---|---|---|---|
| 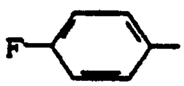 | F | F | - | - | 0 | $CH_3$ |
| 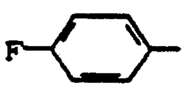 | F | F | - | - | 0 | $C_2H_5$ |

Tabelle 1 (Fortsetzung)

| Ar | $X^1$ | $X^2$ | $X^3$ | $X^4$ | n | R |
|---|---|---|---|---|---|---|
| 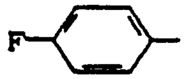 | F | F | - | - | 0 | 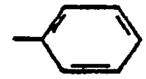 |
| 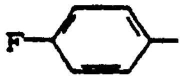 | F | F | - | - | 0 | -$CH_2$ 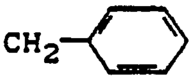 |
| 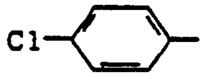 | Cl | Cl | - | - | 0 | 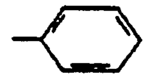 |
| 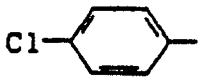 | Cl | Cl | - | - | 0 | -$CH_2$ 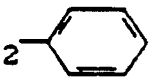 |
| 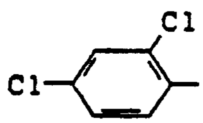 | Cl | Cl | - | - | 0 | $CH_3$ |
| 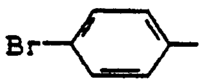 | Cl | Cl | - | - | 0 | $CH_3$ |
| 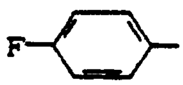 | Cl | Cl | - | - | 0 | $CH_3$ |
| 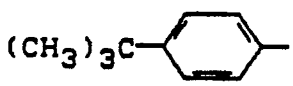 | Cl | Cl | - | - | 0 | $CH_3$ |
| 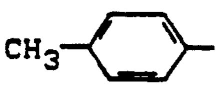 | Cl | Cl | - | - | 0 | $CH_3$ |
| 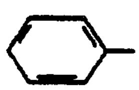 | Cl | Cl | - | - | 0 | $CH_3$ |

Tabelle 1 (Fortsetzung)

| Ar | $X^1$ | $X^2$ | $X^3$ | $X^4$ | n | R |
|---|---|---|---|---|---|---|
| 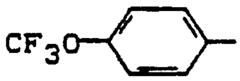 | Cl | Cl | - | - | 0 | $CH_3$ |
| 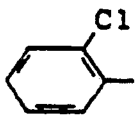 | F | F | - | - | 0 | $CH_3$ |
| 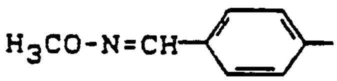 | F | F | - | - | 0 | $CH_3$ |
| 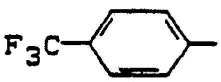 | F | F | - | - | 0 | $CH_3$ |
| 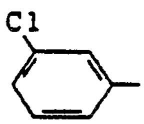 | F | F | - | - | 0 | $CH_3$ |
| 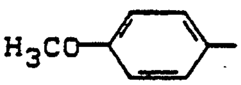 | F | F | - | - | 0 | $CH_3$ |
| 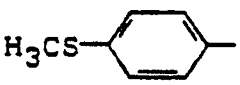 | F | F | - | - | 0 | $CH_3$ |
| 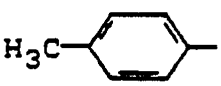 | F | F | - | - | 0 | $CH_3$ |
| 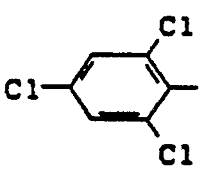 | F | F | - | - | 0 | $CH_3$ |
| 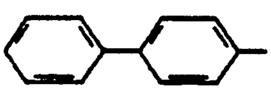 | F | F | - | - | 0 | $CH_3$ |

Tabelle 1 (Fortsetzung)

| Ar | $X^1$ | $X^2$ | $X^3$ | $X^4$ | n | R |
|---|---|---|---|---|---|---|
| 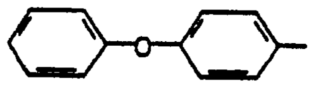 | F | F | - | - | 0 | $CH_3$ |
| 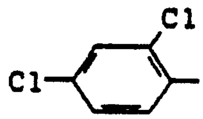 | Cl | F | F | F | 1 | $CH_3$ |
| 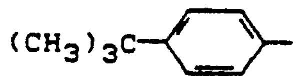 | Cl | F | F | F | 1 | $CH_3$ |
| 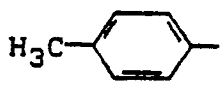 | Cl | F | F | F | 1 | $CH_3$ |
| 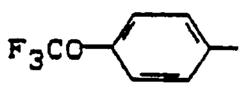 | Cl | F | F | F | 1 | $CH_3$ |

Verwendet man 2-[2-(4-Chlorphenyl)-ethin-1-yl)]-2-(2,2-difluor-1-methyl-cycloprop-1-yl)-oxiran und 1,2,4-Triazol als Ausgangsstoffe, so kann der Verlauf des erfundungsgemäßen Verfahrens (a) durch das folgende Formelschema veranschaulicht werden:

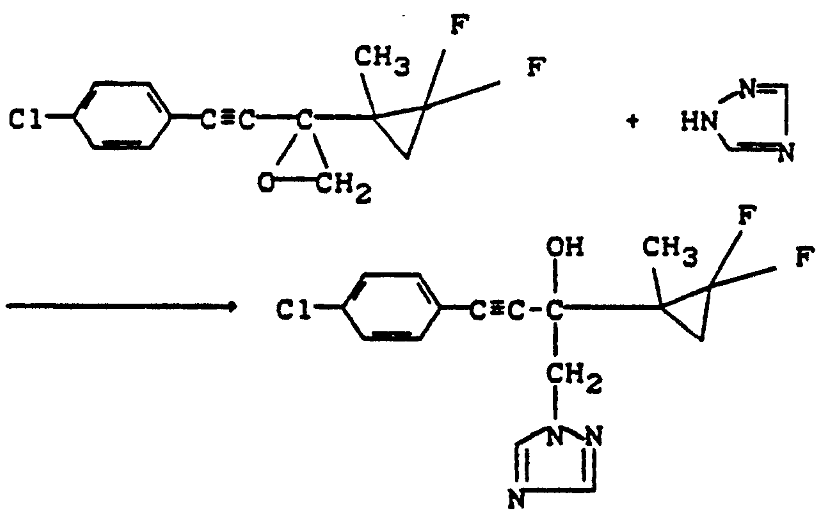

Verwendet man (2,2-Dichlor-1-methyl-cycloprop-1-yl)-(1,2,4-triazol-1-yl-methyl)-keton und 4-Chlorphenylacetylen als Ausgangsstoffe, so kann der Verlauf des erfindungsgemäßen Verfahrens (b) durch das folgende Formelschema veranschaulicht werden:

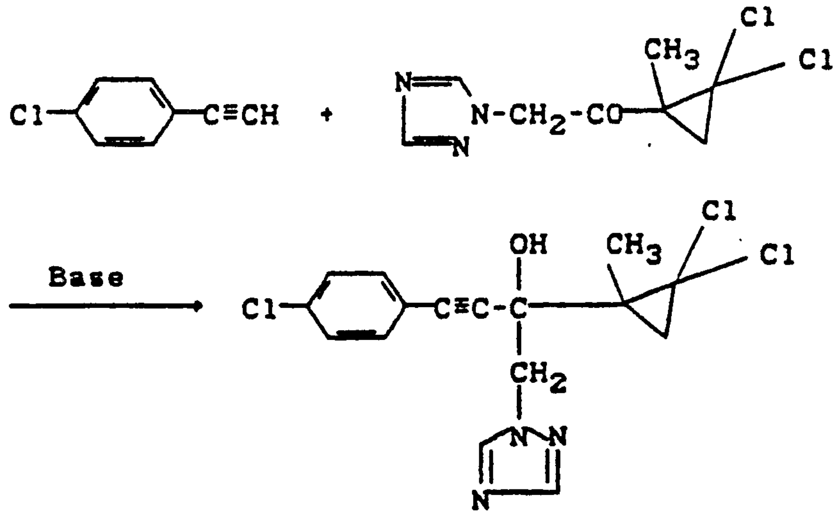


Die bei dem erfindungsgemäßen Verfahren (a) als Ausgangsstoffe benötigten Oxirane sind durch die Formel (II) allgemein definiert. In dieser Formel haben Ar, R, $X^1$, $X^3$, $X^4$ und n vorzugsweise diejenigen Bedeutungen, die bereits im Zusammenhang mit der Beschreibung der erfindungsgemäßen Stoffe der Formel (I) vorzugsweise für diese Reste und diesen Index genannt wurden.

Die Oxirane der Formel (II) sind bisher noch nicht bekannt. Sie lassen sich herstellen, indem man

c) Cycloalkyl-ketone der Formel

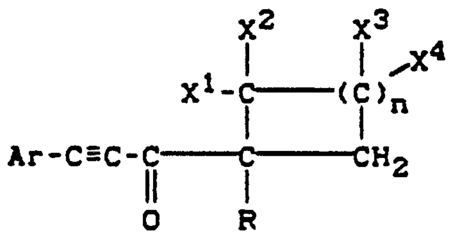
(VI)

in welcher
Ar, R, $X^1$, $X^2$, $X^3$, $X^4$ und n die oben angegebene Bedeutung haben,
mit Dimethylsulfonium-methylid der Formel

$$\overset{\delta\oplus}{(CH_3)_2S}\quad\overset{\delta\ominus}{CH_2} \qquad (VII)$$

in Gegenwart eines Verdünnungsmittels umsetzt.

Die bei der Durchführung des Verfahrens (c) als Ausgangsstoffe benötigten Cycloalkylketone der Formel (VI) sind bisher noch nicht bekannt. Sie lassen sich herstellen, indem man

d) Acetylen-Derivate der Formel

Ar-C≡CH (IV)

in welcher
Ar die oben angegebene Bedeutung hat,
mit Cycloalkyl-carbonsäure-halogeniden der Formel

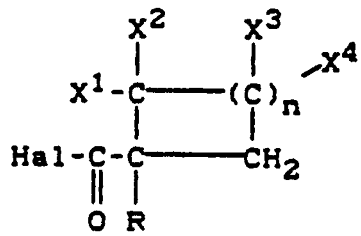

(VIII)

in welcher
R, $X^1$, $X^2$, $X^3$, $X^4$ und n die oben angegebene Bedeutung haben und
Hal für Chlor oder Brom steht, in Gegenwart eines Katalysators und in Gegenwart eines Säurebindemittels sowie in Gegenwart eines Verdünnungsmittels umsetzt.

Die bei dem Verfahren (d) und auch bei dem erfindungsgemäßen Verfahren (b) als Ausgangsstoffe benötigten Acetylen-Derivate sind durch die Formel (IV) allgemein definiert. In dieser Formel hat Ar vorzugsweise diejenigen Bedeutungen, die bereits im Zusammenhang mit der Beschreibung der erfindungsgemäßen Stoffe der Formel (I) vorzugsweise für diesen Rest genannt wurden.

Die Acetylen-Derivate der Formel (IV) sind bekannt oder lassen sich nach prinzipiell bekannten Verfahren herstellen.

Die bei dem Verfahren (d) als Reaktionskomponenten benötigten Cycloalkylcarbonsäure-halogenide der Formel (VIII) sind teilweise bekannt. Sie lassen sich herstellen, indem man Cycloalkylcarbonsäuren der Formel

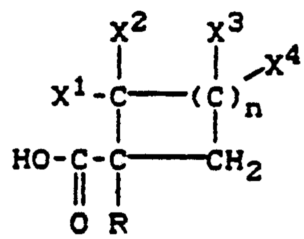

(IX)

in welcher
R, $X^1$, $X^2$, $X^3$, $X^4$ und n die oben angegebene Bedeutung haben,
mit Chlorierungs- oder Bromierungsmitteln, wie Thionylchlorid, Sulfurylchlorid, Phosphortrichlorid, Thionylbromid oder Phosphortribromid, gegebenenfalls in Gegen wart eines Verdünnungsmittels, wie Chloroform oder Tetrachlorkohlenstoff, sowie gegebenenfalls in Gegenwart eines Reaktionsbeschleunigers, wie zum Beispiel Dimethylformamid, bei Temperaturen zwischen 0 °C und 60 °C umsetzt.

Die Cycloalkancarbonsäuren der Formel (IX) sind teilweise bekannt (vgl. J. Amer. Chem. Soc. 71, 493 (1949), J. Org. Chem. 32, 1290 (1967), J. Org. Chem. 51, 1926 (1986), J. Chem. Res. Synop. 1986, 3, 114 und EP-OS 0 043 950).

Die Cycloalkancarbonsäuren der Formel (IX), in denen n für 1 steht, lassen sich herstellen, indem man Acrylsäureester-Derivate der Formel

$CH_2 = \underset{R}{C} -COOR^1$ (X)

in welcher
R die oben angegebene Bedeutung hat und
$R^1$ für Alkyl mit 1 bis 4 Kohlenstoffatomen steht,
mit Ethylen-Derivaten der Formel

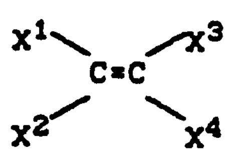

(XI)

in welcher
$X^1$, $X^2$, $X^3$ und $X^4$ die oben angegebene Bedeutung haben,
gegebenenfalls in Gegenwart eines Verdünnungsmittels, sowie gegebenenfalls in Gegenwart von Hydrochinon, bei Temperaturen zwischen -70° C und 150° C und unter Schutzgasatmosphäre unter einem Druck zwischen 1 und 100 bar umsetzt und die so erhaltenen Ester der Formel

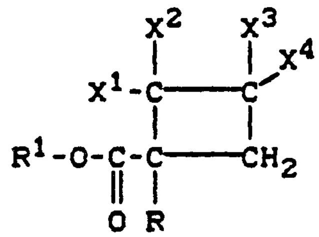

(XII)

in welcher
R, $R^1$, $X^1$, $X^2$, $X^3$ und $X^4$ die oben angegebene Bedeutung haben,
mit Basen, wie zum Beispiel Kaliumhydroxid oder Natriumhydroxid, in Gegenwart eines Verdünnungsmittels, wie zum Beispiel Methanol oder Ethanol, bei Temperaturen zwischen 0° C und 80° C umsetzt und ansäuert.

Die Cycloalkancarbonsäuren der Formel (IX), in denen n für 0 steht, lassen sich herstellen, indem man Vinylcyclopropan-Derivate der Formel

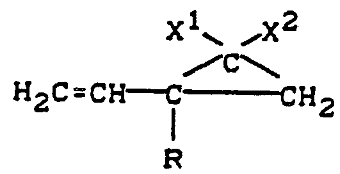

(XIII)

in welcher
R, $X^1$ und $X^2$ die oben angegebene Bedeutung haben,
mit Oxidationsmitteln, wie zum Beispiel Kaliumpermanganat, in Gegenwart eines Verdünnungsmittels, wie zum Beispiel Wasser, bei Temperaturen zwischen 0° C und 50° C umsetzt.

Die Verbindungen der Formel (X), (XI) und (XIII) sind bekannt oder lassen sich nach prinzipiell bekannten Verfahren herstellen.

Bei der Durchführung des Verfahrens (d) kommen als Katalysatoren alle für derartige Umsetzungen übliche Reaktionsbeschleuniger in Betracht. Vorzugsweise verwendbar sind Kupfersalze, wie zum Beispiel Kupfer-(I)-bromid.

Als Säurebindemittel kommen bei der Durchführung des Verfahrens (d) alle für derartige Umsetzungen üblichen Basen in Frage. Vorzugsweise verwendbar sind Alkalihydroxide, wie Natriumhydroxid und Kaliumhydroxid, ferner tertiäre Amine, wie Triethylamin.

Als Verdünnungsmittel können bei der Durchführung des Verfahrens (d) alle üblichen inerten organischen Sol ventien eingesetzt werden. Vorzugsweise verwendbar sind aliphatische oder aromatische Kohlenwasserstoffe, wie Pentan, Hexan, Benzol, Toluol und Xylol.

Die Reaktionstemperaturen können bei der Durchführung des Verfahrens (d) innerhalb eines größeren Bereiches variiert werden. Im allgemeinen arbeitet man bei Temperaturen zwischen 0° C und 150° C, vorzugsweise zwischen 20° C und 120° C.

Das Verfahren (d) wird ebenso wie die anderen erfindungsgemäßen Verfahren im allgemeinen unter Normaldruck durchgeführt. Es ist jedoch jeweils auch möglich, unter erhöhtem oder vermindertem Druck zu arbeiten.

Bei der Durchführung des Verfahrens (d) geht man im allgemeinen so vor, daß man auf 1 Mol an Acetylen-Derivat der Formel (IV) 1 Mol an Cycloalkyl-carbonsäurehalogenid der Formel (VIII) sowie eine katalytische Menge an Reaktionsbeschleuniger einsetzt. Es ist jedoch auch möglich, die eine oder andere Komponente in einem Überschuß zu verwenden. Außerdem ist es zweckmäßig, unter Schutzgasatmosphäre zu arbeiten.

Das bei dem Verfahren (c) als Reaktionskomponente in Betracht kommende Dimethylsulfoniummethylid der Formel (VII) ist bekannt (vgl. Heterocycles 8, 397 (1977)). Es wird bei der obigen Umsetzung

in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (c) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des Verfahrens (c) in einem größeren Bereich variiert werden. Im allgemeinen arbeitet man zwischne 0°C und 100°C, vorzugsweise zwischen 10°C und 60°C.

Bei der Durchführung des Verfahrens (c) setzt man auf 1 Mol an Cycloalkyl-keton der Formel (VI) im allgemeinen 1 bis 3 Mol an Dimethylsulfonium-methylid der Formel (VII) ein. Die Isolierung der Oxirane der Formel (II) erfolgt nach üblichen Methoden.

Das bei der Durchführung des erfindungsgemäßen Verfahrens (a) als Reaktionskomponente benötigte 1,2,4-Triazol der Formel (III) ist eine allgemein bekannte Verbindung der organischen Chemie.

Als Säurebindemittel kommen bei der Durchführung des erfindungsgemäßen Verfahrens (a) alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung des erfindungsgemäßen Verfahrens (a) alle üblichen inerten organischen Solventien in Betracht. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 200°C, vorzugsweise zwischen 50 und 150°C.

Bei der Durchführung des erfindungsgemäßen Verfahrens (a) setzt man auf 1 Mol an Oxiran der Formel (II) im allgemeinen 1 bis 4 Mol an 1,2,4-Triazol der Formel (III) und 1 bis 2 Mol an Säurebindemittel ein. Die Isolierung der Endprodukte erfolgt nach üblichen Methoden.

Die bei der Durchführung des erfindungsgemäßen Verfahrens (b) als Reaktionskomponenten benötigten Azolylmethyl-ketone sind durch die Formel (V) allgemein definiert. In dieser Formel haben R, $X^1$, $X^2$, $X^3$, $X^4$ und n vorzugsweise diejenigen Bedeutungen, die bereits im Zusammenhang mit der Beschreibung der erfindungsgemäßen Verbindungen der Formel (I) vorzugsweise für diese Reste und den Index n genannt wurden.

Die Azolylmethyl-ketone der Formel (V) sind bisher noch nicht bekannt. Sie lassen sich herstellen, indem man

e) Halogenketone der Formel

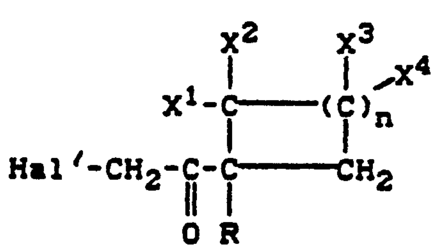

(XIV)

in welcher

R, $X^1$, $X^2$, $X^3$, $X^4$ und n die oben angegebene Bedeutung haben und

Hal′ für Chlor oder Brom steht,

mit 1,2,4-Triazol der Formel

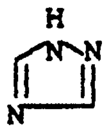

(III)

in Gegenwart eines Säurebindemittels und in Gegenwart eines Verdünnungsmittels umsetzt.

Die bei dem Verfahren (e) als Ausgangsstoffe benötigten Halogenketone der Formel (XIV) lassen sich herstellen, indem man

f) Methyl-cycloalkyl-ketone der Formel

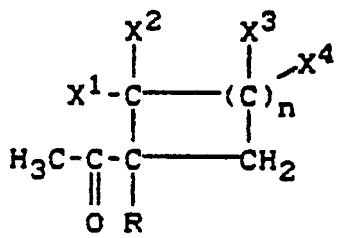

(XV)

in welcher
R, $X^1$, $X^2$, $X^3$, $X^4$ und n die oben angegebene Bedeutung haben,
mit Chlorierungsmitteln oder Bromierungsmitteln in Gegenwart eines Verdünnungsmittels umsetzt.

Die bei dem Verfahren (f) als Ausgangsstoffe benötigten Methyl-cycloalkyl-ketone der Formel (XV) lassen sich herstellen, indem man Cycloalkylcarbonsäuren der Formel

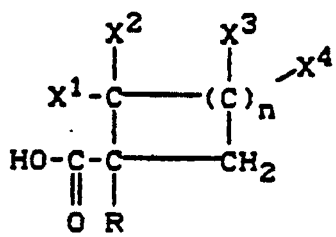

(IX)

in welcher
R, $X^1$, $X^2$, $X^3$, $X^4$ und n die oben angegebene Bedeutung haben,
mit Methyl-lithium in Gegenwart eines Verdünnungsmittels, wie zum Beispiel Diethylether, bei Temperaturen zwischen -80 °C und +20 °C umsetzt.

Als Chlorierungsmittel und Bromierungsmitetl kommen bei dem Verfahren (f) alle für derartige Umsetzungen üblichen Chlorierungs- und Bromierungsreagenzien in Betracht. Vorzugsweise verwendbar sind Sulfurylchlorid, Sulfurylbromid und Brom.

Als Verdünnungsmittel kommen bei dem Verfahren (f) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (f) innerhalb eines bestimmten Bereiches variiert werden. Im allgemeinen arbeitet man bei Temperaturen zwischen -10 °C und +60 °C, vorzugsweise zwischen 0 °C und +40 °C.

Bei der Durchführung des Verfahrens (f) setzt man auf 1 Mol an Methyl-cycloalkyl-keton der Formel (XV) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wäßriger Natriumhydrogencarbonat-Lösung und Wasser wäscht, dann trocknet und einengt.

Als Säurebindemittel und als Verdünnungsmittel kommen bei der Durchführung des Verfahrens (e) alle diejenigen Substanzen in Betracht, die bereits im Zusammenhang mit der Durchführung des erfindungsgemäßen Verfahrens (a) vorzugsweise genannt wurden.

Die Reaktionstemperaturen lassen sich bei der Durchführung des Verfahrens (e) innerhalb eines größeren Bereiches variieren. Im allgemeinen arbeitet man bei Temperaturen zwischen 0 °C und 150 °C,

vorzugsweise zwischen 20° C und 120° C.

Bei der Durchführung des Verfahrens (e) setzt man auf 1 Mol an Halogenketon der Formel (XIV) im allgemeinen 1 bis 3 Mol an 1,2,4-Triazol der Formel (III) sowie 1 bis 2 Mol an Säurebindemittel ein. Die Aufarbeitung erfolgt nach üblichen Methoden. Im allgemeinen geht man so vor, daß man gegebenenfalls nach vorherigem Abfiltrieren von abgeschiedenen Salzen das Reaktionsgemisch einengt, den Rückstand in einem mit Wasser wenig mischbaren organischen Lösungsmittel aufnimmt, die entstehende Lösung wäscht, trocknet und dann einengt.

Als Verdünnungsmittel kommen für die Durchführung des erfindungsgemäßen Verfahrens (b) inerte organische Solventien in Frage. Vorzugsweise verwendbar sind Ether, wie Tetrahydrofuran, Dioxan, Ethylenglykoldiethylether, Diethylenglykoldimethylether und Diethylether, ferner aromatische Kohlenwasserstoffe, wie Benzol, Chlorbenzol, o-Dichlorbenzol und Toluol, außerdem aliphatische Kohlenwasserstoffe, wie Hexan, Cyclohexan und Methylenchlorid, weiterhin Säureamide, wie Dimethylformamid und Hexamethyl-phosphorsäuretriamid, ferner Sulfoxide, wie Dimethylsulfoxid, sowie Nitrile, wie Acetonitril und auch Acetale, wie Butyraldehyd-dibutylacetal, Acetaldehyd-dibutylacetal und Methylethyl-dioxolan.

Als Basen kommen bei der Durchführung des erfindungsgemäßen Verfahrens (b) starke Basen in Betracht. Vorzugsweise verwendbar sind Alkalimetallamide, -hydride und -hydroxide, wie zum Beispiel Natriumamid, -hydroxid oder -hydrid und Kaliumamid, -hydroxid oder -hydrid, ferner Alkalimetallalkoholate wie Kalium-tert.-butylat, außerdem Grignard-Verbindungen, wie Ethylmagnesium-bromid, oder andere metallorganische Verbindungen, wie n-Butyllithium.

Als Phasentransfer-Katalysatoren kommen bei der Durchführung des erfindungsgemäßen Verfahrens (b) vorzugsweise Ammonium- oder Phosphonium-Verbindungen in Betracht, wie zum Beispiel Tetrabutylammoniumbromid oder -chlorid und Triethyl-benzyl-ammoniumchlorid.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen -80° C und +80° C, vorzugsweise zwischen -10° C und +40° C. Bei Verwendung eines Phasentransfer-Katalysators arbeitet man im allgemeinen zwischen 40° C und 150° C, vorzugsweise zwischen 70° C und 120° C.

Bei der Durchführung des erfindungsgemäßen Verfahrens (b) setzt man die Reaktionskomponenten vorzugsweise in äquimolaren Mengen ein. Die Aufarbeitung und die Isolierung der Endprodukte erfolgt nach üblichen Methoden.

Die nach den erfindungsgemäßen Verfahren (a) und (b) erhältlichen Stoffe der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden:

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;

Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreidekrankheiten, wie Erysiphe graminis, ferner Reiskrankheiten, wie Pyricularia oryzae, und von Rostkrankheiten, wie Uromyces appendiculatus.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokos nußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmenge kann je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den nachfolgenden Beispielen hervor.

Herstellungsbeispiele

Beispiel 1

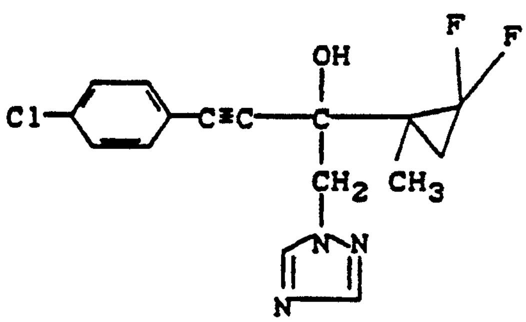

(I-1)

In ein Gemisch aus 0,9 g (0,013 Mol) 1,2,4-Triazol, 1,8 g (0,013 Mol) gemahlenem Kaliumcarbonat und 30 ml Acetonitril werden bei Raumtemperatur unter Rühren 3,5 g 2-[2-(4-Chlorphenyl)-ethin-1-yl)]-2-(2,2-difluor-1-methyl-cycloprop-1-yl)-oxiran gegeben. Das Gemisch wird 8 Stunden unter Rückfluß erhitzt, dann abgekühlt, mit Wasser verdünnt und dreimal mit Methylenchlorid extrahiert. Die vereinigten organischen Phasen werden mit Wasser gewaschen, dann getrocknet und unter vermindertem Druck eingeengt. Man erhält auf diese Weise 3,8 g eines dunklen Öles, das durch Säulenchromatographie (Petrolether/Essigester = 2:1) an Kieselgel gereinigt wird. Es verbleiben 2,1 g (48 % der Theorie) an 1-(4-Chlorphenyl)-3-(2,2-difluor-1-methyl-cycloprop-1-yl)-4-(1,2,4-triazol-1-yl)-but-1-in-3-ol in Form einer Festsubstanz vom Schmelzpunkt 113-115 °C.

Herstellung von Ausgangssubstanzen

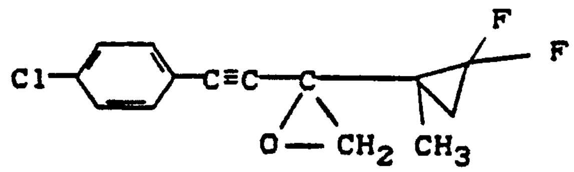

(II-1)

Ein Gemisch aus 13 g Dimethylsulfid und 4,8 g Dimethylsulfat wird zunächst 2 Stunden bei Raumtemperatur gerührt und dann mit 18 ml tert.-Butanol sowie mit 8,9 g [2-(4-Chlorphenyl)-ethin-1-yl]-(2,2-difluor-1-methyl-cycloprop-1-yl)-keton versetzt. Man rührt 30 Minuten bei Raumtemperatur, kühlt auf 10 °C ab und tropft unter Rühren innerhalb von einer Stunde eine Lösung von 4,5 g Kalium-tert.-butylat in 32 ml tert.-Butanol hinzu. Das Reaktionsgemisch wird noch 3 Stunden bei 10 °C nachgerührt und dann durch Abziehen des Verdünnungsmittels unter vermindertem Druck eingeengt. Man nimmt den Rückstand in Methylenchlorid auf, wäscht dreimal mit Wasser, trocknet und engt erneut unter vermindertem Druck ein. Es verbleiben 6,4 g (68 % der Theorie) an 2-[2-[4-Chlorphenyl)-ethin-1-yl]-2-(2,2-difluor-1-methyl-cycloprop-1-yl)-oxiran in Form eines Öles, das ohne zusätzliche Reinigung weiter umgesetzt wird.

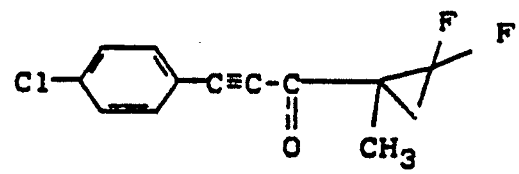

(VI-1)

Ein Gemisch aus 10,1 g (0,1 Mol) Triethylamin, 1,4 g (0,01 Mol) Kupfer-(I)-bromid und 55 ml Toluol wird mit 13,7 g (0,1 Mol) p-Chlorphenyl-acetylen versetzt und 30 Minuten bei Raumtemperatur unter Argon gerührt. Nach dem Erhitzen des Reaktionsgemisches auf 55 °C werden 15,5 g (0,1 Mol) 1-Methyl-2,2-difluorcyclopropancarbonsäurechlorid zugetropft. Danach wird 8 Stunden bei 90 °C gerührt, dann abgekühlt und filtriert. Das Filtrat wird nacheinander mit verdünnter, wäßriger Salzsäure und Wasser gewaschen und unter vermindertem Druck eingeengt. Es verbleiben 19,2 g eines dunklen Öles, das im Kugelrohr destilliert wird. Bei 0,1 mbar und einer Manteltemperatur von 120 °C geht ein gelbes Öl über, das beim Stehen erstarrt. Man erhält 8,4 g (33 % der Theorie) an [2-(4-Chlorphenyl)-ethin-1-yl]-(2,2-difluor-1-methyl-cycloprop-1-yl)-keton vom Schmelzpunkt 67-68 °C.

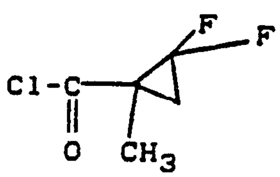

(VIII-1)

Ein Gemisch aus 250 g (1,8 Mol) 2,2-Difluor-1-methyl-cyclopropancarbonsäure und 700 ml Thionylchlorid wird in einer Destillationsapparatur langsam erhitzt, wobei zuerst überschüssiges Thionylchlorid und dann das gewünschte Produkt übergeht. Man erhält auf diese Weise 215 g (77 % der Theorie) an 2,2-Difluor-1-methyl-cyclopropancarbonsäurechlorid in Form einer Flüssigkeit mit dem Siedepunkt 121-122 °C.

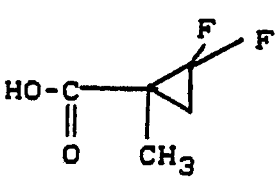

(IX-1)

840 g (7,12 Mol) 2,2-Difluor-1-methyl-1-vinyl-cyclopropan in 10 l Wasser werden portionsweise mit 2,3 kg (14,47 Mol) Kaliumpermanganat versetzt. Man läßt 36 Stunden rühren, filtriert von Braunstein ab und wäscht gut mit Wasser nach. Das Filtrat wird mit konzentrierter Salzsäure sauer gestellt und mit Dichlormethan extrahiert. Nach dem Trocknen der organischen Phase wird das Lösungsmittel im Vakuum entfernt und der Rückstand destilliert.

Man erhält so 750 g (77 % der Theorie) 2,2-Difluor-1-methylcyclopropancarbonsäure vom Schmelzpunkt 59-61 °C.

Beispiel 2

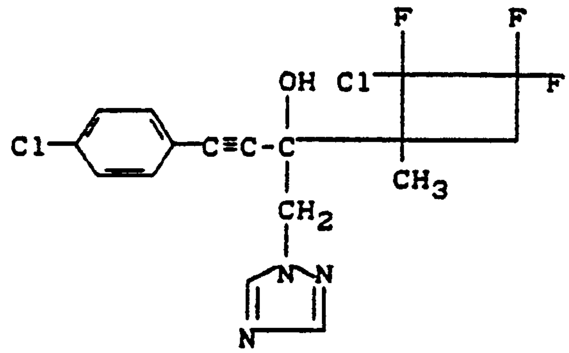

(I-2)

In ein Gemisch aus 6,4 g 1,2,4-Triazol, 12,6 g gemahlenem Kaliumcarbonat und 300 ml Acetonitril werden bei Raumtemperatur unter Rühren 31,8 g 2-[2-(4-Chlor phenyl)-ethin-1-yl]-2-(2,3,3-trifluor-2-chlor-1-methyl-cyclobut-1-yl)-oxiran gegeben. Das Reaktionsgemisch wird 12 Stunden unter Rückfluß erhitzt, dann abgekühlt und filtriert. Das Filtrat wird unter vermindertem Druck eingeengt und der verbleibende Rückstand wird durch Säulenchromatographie (Essigester) an Kieselgel gereinigt. Man erhält 29 g (75,5 % der Theorie) an 1-(4-Chlorphenyl)-3-(2,3,3-trifluor-2-chlor-1-methyl-cyclobut-1-yl)-4-(1,2,4-triazol-1-yl)-but-1-in-3-ol in Form eines zähen Öles.

Herstellung der Ausgangssubstanzen

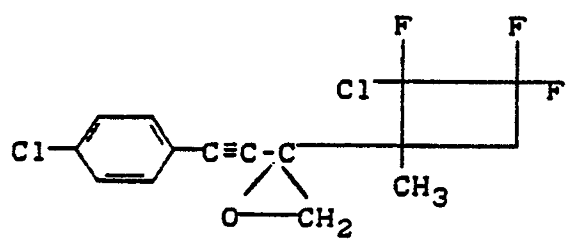

(II-2)

Ein Gemisch aus 37,2 g Dimethylsulfid und 13,2 g Dimethylsulfat wird zunächst 2 Stunden bei Raumtemperatur gerührt und dann mit 25 ml tert.-Butanol sowie mit 16,05 g [2-(4-Chlorphenyl)-ethin-1-yl]-(2,3,3-trifluor-2-chlor-1-methyl-cyclobut-1-yl)-keton versetzt. Man rührt 30 Minuten bei Raumtemperatur, kühlt auf 10 °C ab und tropft unter Rühren innerhalb von 1,25 Stunden eine Lösung von 6,4 g Kalium-tert.-butylat in 45 ml tert.-Butanol hinzu. Das Reaktionsgemisch wird noch 3 Stunden bei 10 °C nachgerührt und dann durch Abziehen des Verdünnungsmittels unter vermindertem Druck eingeengt. Man nimmt den Rückstand in Methylenchlorid auf, wäscht drei mal mit Wasser, trocknet und engt erneut unter vermindertem Druck ein. Es verbleiben 31,8 g (95 % der Theorie) an 2-[2-(4-Chlorphenyl)-ethin-1-yl]-2-(2,3,3-trifluor-2-chlor-1-methyl-cyclobut-1-yl)-oxiran in Form eines Öles, das ohne zusätzliche Reinigung weiter umgesetzt wird.

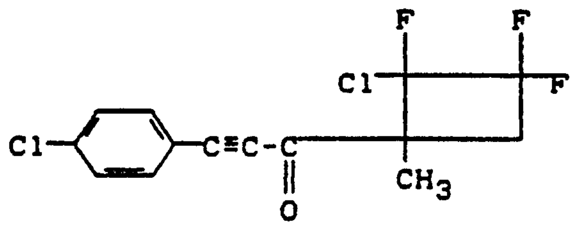

(VI-2)

Ein Gemisch aus 30,3 g (0,3 Mol) Triethylamin, 4,5 g Kupfer-(I)-bromid und 250 ml Toluol wird mit 41 g p-Chlorphenyl-acetylen versetzt und unter Argon 30 Minuten bei Raumtemperatur gerührt. Nach dem Erhitzen des Reaktionsgemisches auf 50 °C werden 66,3 g 1-Methyl-2-chlor-2,3,3-trifluor-cyclobutan-1-carbonsäurechlorid zugetropft. Danach wird 12 Stunden unter Rühren auf 85 °C erhitzt, dann abgekühlt und filtriert. Das Filtrat wird nacheinander mit verdünnter, wäßriger Salzsäure und Wasser gewaschen, dann getrocknet und unter vermindertem Druck eingeengt. Der verbleibende Rückstand wird mit Hexan gewaschen. Man erhält 65 g (67,5 % der Theorie) an 2-[2-(4-Chlorphenyl)-ethin-1-yl]-2-(2,3,3-trifluor-2-chlor-1-methyl-cyclobut-1-yl)-keton in Form einer Festsubstanz vom Schmelzpunkt 69 °C.

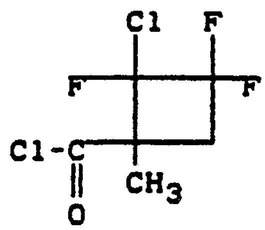

(VIII-2)

Ein Gemisch aus 91,2 g (0,45 Mol) 1-Methyl-2,3,3-trifluor-2-chlor-cyclobutan-1-carbonsäure und 450 ml Chloroform wird mit einem Tropfen Dimethylformamid versetzt. Danach tropft man bei 25 - 30 °C unter Rühren 80,3 g (0,675 Mol) Thionylchlorid hinzu und erhitzt unter Rühren zum Sieden. Nach 6 Stunden wird abgekühlt, das Lösungsmittel abgezogen und der Rückstand einer fraktionierten Destillation unter vermindertem Druck unterworfen. Man erhält 75 g (75 % der Theorie) an 1-Methyl-2-chlor-2,3,3-trifluor-cyclobutan-1-carbonsäurechlorid in Form einer Flüssigkeit mit einem Siedepunkt von 65 - 70 °C bei 20 mbar.

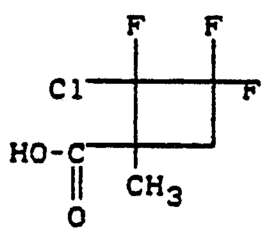

(IX-2)

Zu einer Lösung von 127,3 g Kaliumhydroxid in 540 ml Methanol werden bei 20 - 25 °C unter Rühren innerhalb von 15 Minuten 108,3 g (0,5 Mol) 1-Methyl-2-chlor-2,3,3-trifluor-cyclobutan-1-carbonsäuremethylester hinzugetropft. Es wird weitere 12 Stunden bei 20 - 25 °C gerührt und dann das Methanol unter vermindertem Druck teilweise abgezogen. Man verdünnt den Rückstand mit Wasser und extrahiert mit Methylenchlorid. Die wäßrige Phase wird mit verdünnter Salzsäure angesäuert und mit Methylenchlorid extrahiert. Die vereinigten organischen Phasen werden getrocknet und unter vermindertem Druck eingeengt. Man erhält 93,3 g eines Öles, das zu 99 % aus 1-Methyl-2-chlor-2,3,3-trifluor-cyclobutan-1-carbonsäure besteht. Die Ausbeute errechnet sich danach zu 90 % der Theorie.

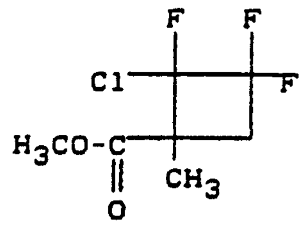

(XII-1)

In einem Autoklaven werden 174 g (1,74 Mol) Methacrylsäure-methylester und 1,5 g Hydrochinon vorgelegt und bei -70° c mit 200 g (1,74 Mol) Trifluorchlorethylen versetzt. Nach dem Aufpressen von 7 bar Stickstoff wird auf 140° C erwärmt. Der sich bei dieser Temperatur einstellende Druck wird durch weiteres Aufpressen von Stickstoff auf 28 bar erhöht. Man hält 17 Stunden auf 140° C, läßt dann abkühlen, entspannt und destilliert. Dabei erhält man 203 g (54 % der Theorie) an 1-Methyl-2-chlor-2,3,3-trifluor-cyclobutan-1-carbonsäuremethylester in Form einer Flüssigkeit vom Siedepunkt 57° C bei 17 mbar.

Nach den in den Beispielen 1 und 2 angegebenen Methoden werden auch die in der folgenden Tabelle 2 aufgeführten Verbindungen der Formel (I) hergestellt.

**Tabelle 2**

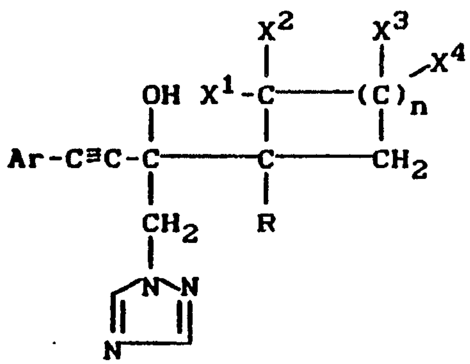

(I)

| Beisp. Nr. | Verb. Nr. | Ar | $X^1$ | $X^2$ | $X^3$ | $X^4$ | n | R | Physik. Konstante |
|---|---|---|---|---|---|---|---|---|---|
| 3 | I-3 | Cl 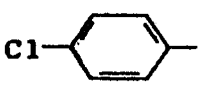 | Cl | Cl | - | - | 0 | $CH_3$ | Harz *) |
| 4 | I-4 | 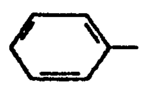 | Cl | F | F | F | 1 | $CH_3$ | Harz **) |
| 5 | I-5 | F 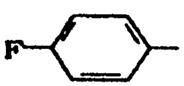 | Cl | F | F | F | 1 | $CH_3$ | Harz |

**Tabelle 2** (Fortsetzung)

| Beisp. Nr. | Verb. Nr. | Ar | $X^1$ | $X^2$ | $X^3$ | $X^4$ | n | R | Physik. Konstante |
|---|---|---|---|---|---|---|---|---|---|
| 6 | I-6 | 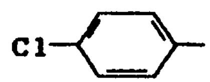 | Cl | F | - | - | 0 | $CH_3$ | Harz |
| 7 | I-7 | 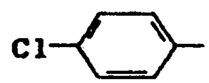 | F | H | - | - | 0 | $CH_3$ | Harz |
| 8 | I-8 | 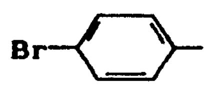 | Cl | F | F | F | 1 | $CH_3$ | Fp. 78° C |

*) Die Verbindung (I-3) ist durch ihr $^1$H-NMR-Spektrum ($CDCl_3$) als Gemisch von 2 Diastereomeren-Paaren charakterisiert.

1,25 und 1,9 ppm Cyclopropan - $CH_2$
1,5 und 2,4 ppm

1,7 ppm (d) $CH_3$

4,0 ppm OH

4,7 und 5,3 ppm (m) $CH_2$

7,3 ppm Aromat

7,9 - 8,3 ppm (dd) Triazol-H

**) Die Verbindung (I-4) enthält 3 asymetrische Kohlenstoffatome: Man erhält daher ein Isomerengemisch, welches durch das $^1H$-NMR-Spektrum ($CDCl_3$) charakterisiert wurde:

| | | | | |
|---|---|---|---|---|
| 1,65 | | | ppm | $CH_3$-Gruppe |
| 2,3 | - | 3,6 | ppm | Cyclobutan-$CH_2$ |
| 4,5 | | | ppm | -$CH_2$-(Triazol) |
| 7,2 | - | 7,4 | ppm | 5H(Aromat) |
| 7,95 | und | 8,3 | ppm | 2H(Triazol) |

Nach den in den Beispielen 1 und 2 angegebenen Methoden werden auch die in der folgenden Tabelle 3 aufgeführten Verbindungen der Formel (VI) hergestellt.

**Tabelle 3**

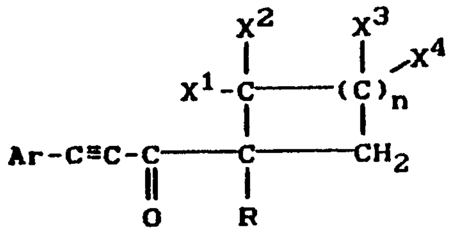

(VI)

| Beisp. Nr. | Verb. Nr. | Ar | $X^1$ | $X^2$ | $X^3$ | $X^4$ | n | R | Physik. Konstante |
|---|---|---|---|---|---|---|---|---|---|
| 6 | VI-3 | 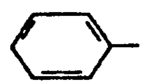 | Cl | F | F | F | 1 | $CH_3$ | $n_D^{20} = 1,5191$ |
| 7 | VI-4 | 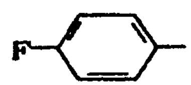 | Cl | F | F | F | 1 | $CH_3$ | Kp= 160°C/2,5 mbar |
| 8 | VI-5 | 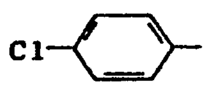 | Cl | Cl | - | - | 0 | $CH_3$ | Fp = 87°C |
| 9 | VI-6 | 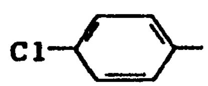 | Cl | F | - | - | 0 | $CH_3$ | Fp = 65°C |

In den folgenden Verwendungsbeispielen wurde die nachstehend aufgeführte Verbindung als Vergleichssubstanz eingesetzt:

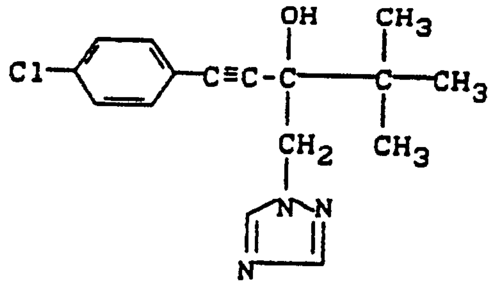

= (A)

(Bekannt aus EP-OS 0 052 424).

Beispiel A

Erysiphe-Test (Gerste) / protektiv

Lösungsmittel: 100 Gewichtsteile Dimethylformamid
Emulgator 0,25 Gewichtsteile Alkyarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man june Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) eine wesentlich bessere Wirkung als die Vergleichssubstanz (A).

Beispiel B

Uromyces-Test (Buschbohne) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22° C und 100 % relativer Luftfeuchtigkeit.

Die Pflanzen werden dann unter intensiver Belichtung für 9 Tage bei 20 bis 22° C und einer relativen Luftfeuchtigkeit von 70 bis 80 % im Gewächshaus aufgestellt.

10 Tage nach der Inokulation erfolgt die Auswertung.

In diesem Test zeigt die erfindungsgemäße Verbindung (I-1) eine bessere Wirksamkeit als die Vergleichssubstanz (A).

Beispiel C

Pyricularia-Test (Reis) / protektiv

Lösungsmittel: 12,5 Gewichtsteile Aceton
Eumulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalles.

In diesem Test zeigen die erfindungsgemäßen Verbindungen (I-1) und (I-2) eine wesentlich bessere Wirksamkeit als die Vergleichssubstanz (A).

## Ansprüche

1. Hydroxyalkinyl-azolyl-Derivate der Formel

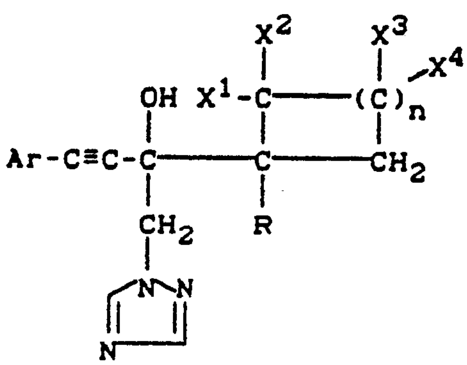

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht
und
n für 0 oder 1 steht,
sowie deren Säureadditions-Salze und Metallsalz-Komplexe.

2. Verfahren zur Herstellung von Hydroxyalkinylazolyl-Derivaten der Formel

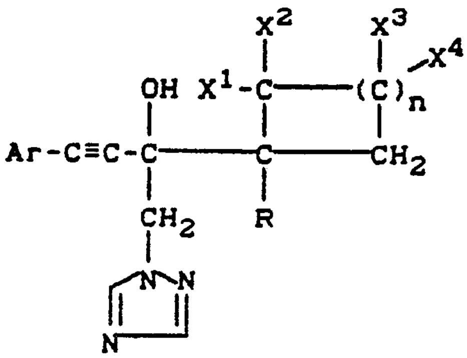

(I)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht
und
n für 0 oder 1 steht,
sowie von deren Säureadditions-Salzen und Metallsalz-Komplexen, dadurch gekennzeichnet, daß man
a) Oxirane der Formel

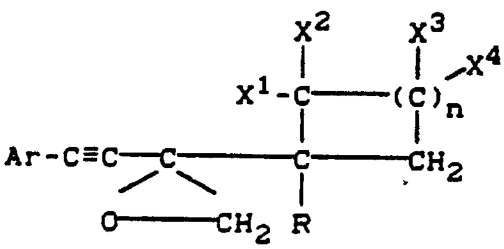

(II)

in welcher
Ar, R, $X^1$, $X^2$, $X^3$, $X^4$ und n die oben angegebene Bedeutung haben,
mit 1,2,4-Triazol der Formel

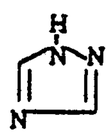

(III)

in Gegenwart eines Säurebindemittels und in Gegenwart eines Verdünnungsmittels umsetzt,
oder
b) Acetylen-Derivate der Formel
Ar - C ≡ CH (IV)
in welcher
Ar die oben angegebene Bedeutung hat,
mit Azolylmethyl-ketonen der Formel

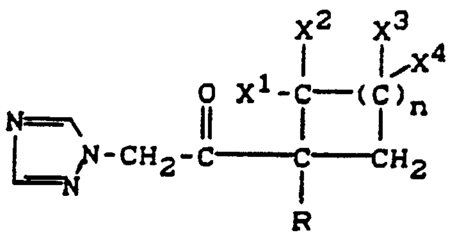

(V)

in welcher
R, $X^1$, $X^2$, $X^3$, $X^4$ und n die oben angegebene Bedeutung haben,
in Gegenwart einer Base und in Gegenwart eines Verdünnungsmittels sowie gegebenenfalls in Gegenwart eines Phasentransfer-Katalysators umsetzt,
und gegebenenfalls anschließend an die so erhaltenen Verbindungen der Formel (I) eine Säure oder ein Metallsalz addiert.

3. Fungizide Mittel, gekennzeichnet, durch einen Gehalt an mindestens einem Hydroxyalkinyl-azolyl-Derivat der Formel (I) gemäß Anspruch 1 bzw. einem Säureadditions-Salz oder Metallsalz-Komplex eines Hydroxyalkinyl-azolyl-Derivates der Formel (I).

4. Verwendung von Hydroxyalkinyl-azolyl-Derivaten der Formel (I) gemäß Anspruch 1 bzw. von deren Säureadditions-Salzen oder Metallsalz-Komplexen zur Bekämpfung von Pilzen.

5. Verfahren zur Bekämpfung von Pilzen, dadurch gekennzeichnet, daß man Hydroxyalkinyl-azolyl-Derivate der Formel (I) gemäß Anspruch 1 bzw. deren Säureadditions-Salze oder Metallsalz-Komplexe auf die Pilze und/oder deren Lebensraum ausbringt.

6. Verfahren zur Herstellung von fungiziden Mitteln, dadurch gekennzeichnet, daß man Hydroxyalkinyl-azolyl-Derivate der Formel (I) gemäß Anspruch 1 bzw. deren Säureadditions-Salze oder Metallsalz-Komplexe mit Streckmitteln und/oder oberflächenaktiven Stoffen vermischt.

7. Oxirane der Formel

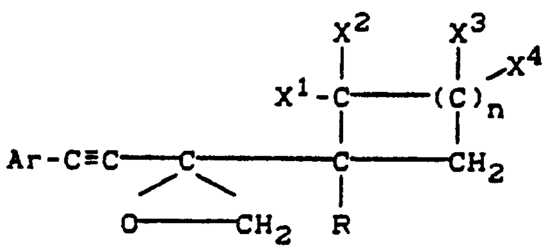

(II)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht,
und
n für 0 oder 1 steht.

8. Verfahren zur Herstellung von Oxiranen der Formel

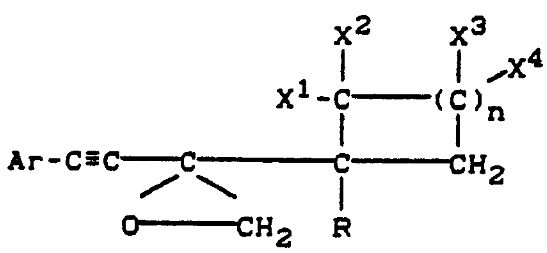

(II)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht
und
n für 0 oder 1 steht,
dadurch gekennzeichnet, daß man
c) Cycloalkyl-ketone der Formel

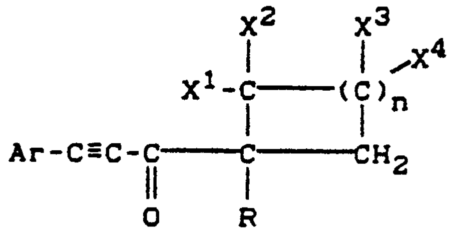

(VI)

in welcher
Ar, R, $X^1$, $X^2$, $X^3$, $X^4$ und n die oben angegebene Bedeutung haben,
mit Dimethylsulfonium-methylid der Formel

$$(CH_3)_2\overset{\delta\oplus}{S}\quad \overset{\delta\ominus}{CH_2} \qquad (VII)$$

in Gegenwart eines Verdünnungsmittels umsetzt.

9. Cycloalkyl-ketone der Formel

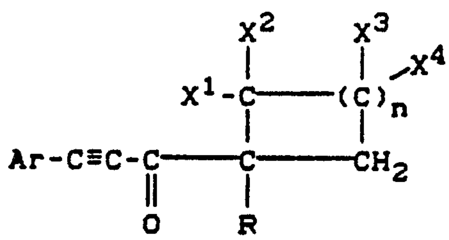

(VI)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht
und
n für 0 oder 1 steht.

10. Verfahren zur Herstellung von Cycloalkylketonen der Formel

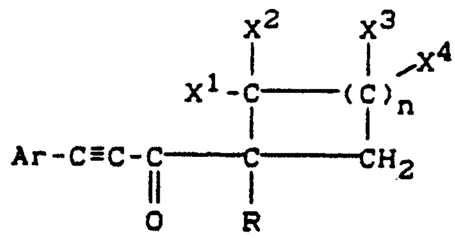

(VI)

in welcher
Ar für gegebenenfalls substituiertes Aryl steht,
R für Alkyl, gegebenenfalls substituiertes Aryl oder gegebenenfalls substituiertes Aralkyl steht,
$X^1$ für Halogen steht,
$X^2$ für Wasserstoff oder Halogen steht,
$X^3$ für Wasserstoff oder Halogen steht,
$X^4$ für Wasserstoff oder Halogen steht
und
n für 0 oder 1 steht,
dadurch gekennzeichnet, daß man
d) Acetylen-Derivate der Formel
$Ar - C \equiv CH$ (IV)
in welcher
Ar die oben angegebene Bedeutung hat,
mit Cycloalkyl-carbonsäure-halogeniden der Formel

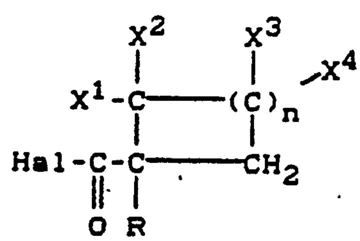

(VIII)

in welcher
R, $X^1$, $X^2$, $X^3$, $X^4$ und n die oben angegebene Bedeutung haben und
Hal für Chlor oder Brom steht,
in Gegenwart eines Katalysators und in Gegenwart eines Säurebindemittels sowie in Gegenwart eines Verdünnungsmittels umsetzt.